# EUROPEAN PATENT APPLICATION

(11) **EP 3 257 501 A1**
(43) Date of publication of application: **20.12.2017**
(21) Application number: 16174466.9
(22) Date of filing: 14.06.2016
(51) Int. Cl.: A61K 9/48, A61K 9/50, A61K 31/4164, A61P 1/00

(54) **MULTIPLE UNIT DOSAGE FORM COMPRISING A CORE WITH INDIVIDUAL CORE UNITS COVERED BY A MUCOADHESIVE MATERIAL, AND AN ENTERIC CORE COATING**

(71) Applicant: Tillotts Pharma AG, 4310 Rheinfelden (CH)
(72) Inventor: The designation of the inventor has not yet been filed
(74) Representative: Best, Michael

(57) **Abstract**

The present invention relates to a multiple unit dosage form comprising a core, wherein the core comprises a plurality of individual units, each unit comprising a pharmaceutically active ingredient and each unit being covered with a mucoadhesive material, and wherein the multiple unit dosage form further comprises an enteric core coating layer. The invention furthermore relates to the use of said multiple unit dosage form in the treatment of inflammatory disorders, especially for the gastrointestinal tract.

## Description

The present invention relates to a multiple unit dosage form comprising a core, wherein the core comprises a plurality of individual units, each unit comprising a pharmaceutically active ingredient and each unit being covered with a mucoadhesive material, wherein the multiple unit dosage form further comprises an enteric coating layer on the core. The invention furthermore relates to the said multiple unit dosage form for use in the treatment of Crohn's disease and/or ulcerative colitis.

The local treatment of bowel diseases, such as Crohn's disease and ulcerative colitis, or the targeting of drugs to the intestine and in particular to the colon for systemic administration is highly challenging because conventional dosage forms rapidly release the drug in the upper gastrointestinal tract. Upon absorption into the blood stream, the drug is distributed throughout the human body, resulting in potentially severe side effects. In addition, the drug concentration at the site of action, such as the inflamed colon, is low, leading to low therapeutic efficacies. To overcome these restrictions, drug release from the dosage form should ideally be suppressed in the stomach and small intestine, but set on as soon as the target site is reached.

Different approaches have been described in the prior art to allow for site- or target-specific drug delivery to the small and/or large intestine upon oral administration. Generally, a drug reservoir is surrounded by a film coating, which is poorly permeable to the drug in the upper gastrointestinal tract, but becomes permeable as soon as the target site is reached. Furthermore, drug release might start right after oral administration at a rate which is sufficiently small in order to assure that drug is still present in the dosage form once the target site is reached.

Release of drugs in the colon typically requires a different approach than the release in the small intestine. The colon is susceptible to a number of disease states, including inflammatory diseases, irritable bowel syndrome, constipation, diarrhea, infection and carcinoma. In such conditions, drug targeting to the colon would maximize the therapeutic effectiveness of the treatment. The colon can also be utilized as a portal for the entry of drugs into the systemic circulation and therefore can be effective in the treatment of diseases outside the colon.

Various formulations have been developed for intestinal and in particular colonic drug delivery, including pro-drugs as well as formulated dosage forms, with the latter being more popular since the concept once proofed can be applied to other active agents. Examples for devices and dosage forms for controlled release of an active agent to the colon can e.g. be found in US 4,627,851, WO 83/00435 and WO 2007/122374.

EP 2 018 159 describes a delayed release drug formulation for delivery of a drug to the colon comprising a single unit core and coating for the core. The core comprises a drug and the coating comprises a mixture of a polysaccharide material such as starch, and a further material selected from an acrylate polymer or a cellulose polymer or a polyvinyl-based polymer. The combination prevents swelling of materials and allows a relatively quick release of the drug in the colon.

WO2008/135090 discloses a solid dosage form comprising an inner coating located between a single unit core containing a pharmaceutically active ingredient and an outer enteric coating. The material of the inner coating is preferably made of a partially neutralized anionic (meth)acrylate co-polymer, and the outer coating is preferably made of an anionic (meth)acrylate. The coating layer allows release on the entry into the small intestine where the pH is around 5.5 to 7.0.

Delayed release drug formulations for delivery of a drug to the colon are also described in WO2013/164315 and WO2013/164316. These formulations comprise a single unit core comprising the drug, and a coating for the core, comprising an inner layer and an outer layer. The outer layer comprises a mixture of a first polymeric material which is susceptible to attack by colonic bacteria and a second polymeric material which has a pH threshold at about 5 or above. The first polymeric material represents a bacteria trigger responding to the bacteria in the colon, which is preferably starch. The second polymeric material represents a pH trigger responding to the pH conditions in the intestine, which is preferably an anionic co-polymer of a (meth)acrylic acid and a (meth)acrylic acid alkyl ester. The inner layer comprises a third polymeric material which is soluble in intestinal fluid or gastrointestinal fluid. A related delayed release drug formulation is also described in WO2015/062640.

The formulations described above are designed for site-specific release of a drug in the intestine. On the other hand, the site-specific release in the intestine, and in particular in the colon, is extremely sensitive to inter-individual variability. The release is particularly dependent on the individual environment in the gastrointestinal tract, like the pH, the transit time and the bacterial flora. The pH value in the gastrointestinal tract is usually subject to fluctuations, which means that the pH trigger in the above described formulations might not necessarily respond efficiently in the colon but in a different region of the intestine or later in the distal regions of the colon. The residence time of the formulation in the target region, i.e. the colon might thereby be diminished making it difficult to ensure and to reproduce the full therapeutic effect. Also, patients with chronic inflammation of the intestine often show a misbalance in the microbiome, which means that the bacterial trigger might not respond promptly in the colon either. In consequence, efficient release of the drug at the desired target, i.e. the colon, may be diminished, resulting in a reduced therapeutic efficiency of the drug and/or reduced reproducibility of the therapeutic effect, and potentially increased side effects. Accordingly, there is a need for strategies that allow increasing the residence time of the formulation in the intestine, in particular in the colon of the patient in order to overcome the above deficiencies and to improve the therapeutic effect in the target region, in particular in the colon of the patient. This problem is not solved by the formulations as described above.

Lamprecht et al. (European Journal of Pharmaceutics and Biopharmaceutics 2012, 81, 379-385) describe a study wherein bioadhesive pellets loaded with 5-aminosalicyclic acid and chitosan were investigated with respect to their potential in the treatment of inflammatory bowel disease. These pellets were further coated with an Eudragit® FS layer in order to allow controlled drug delivery in the colon. However, the particles in the Lamprecht study were not provided in a dosage form suitable for administration to a human patient, let alone to a patient with increased peristalsis of the digestive system and/or chronic inflammation of the intestine.

Thus, particularly in view of patients with increased peristalsis of the digestive system and/or chronic inflammation of the intestine, there is still a need for dosage forms that provide efficient and reproducible site-specific release of a pharmaceutically active ingredient in the intestine, in particular in the colon of the patient.

It has now surprisingly been found that when providing a pharmaceutically active ingredient in a multiple unit dosage form which comprises a core of individual units, each comprising the pharmaceutically active ingredient and each being covered by a mucoadhesive material, wherein the core is further coated with an enteric coating layer, site-specific release of the active ingredient in the colon, especially in patients with increased peristalsis and chronic inflammation of the intestine, can significantly be improved.

Thus, the present invention relates to a multiple unit dosage form comprising
(a) a core and
(b) a core coating which is a coating which completely covers the core
wherein the core comprises a plurality of individual units, each unit comprising
(a1) at least one pharmaceutically active ingredient,
   and wherein each unit is covered by
(a2) at least one mucoadhesive material,
wherein the core coating (b) comprises at least one coating layer, namely an outer coating layer (b1),
wherein the outer coating layer (b1) of the core coating (b) is an enteric coating layer which is substantially insoluble in the stomach of a patient and soluble in the intestine of a patient.

Preferred embodiments of the multiple unit dosage of the present invention are defined in the subclaims and are further explained in the following description of the present invention.

The multiple unit dosage form of the present invention allows site (target)-specific release of the active ingredient in the intestine, preferably the colon of a patient.

The outer enteric coating layer (b1) of the core is designed for intestinal, preferably colonic targeting, and prevents release of the active ingredient in the stomach of the patient, i.e. before the dosage form reaches the intestine, preferably before it reaches the terminal ileum, most preferably before it enters the colon. Thereby, systemic availability of the active ingredient is reduced, which means that a lower dose of the pharmaceutically active ingredient can be used in the dosage form, and, in consequence, side effects are diminished. On the other hand, pharmaceutically active ingredients which are extensively metabolized in the small intestine or are substrates for efflux transporters can be delivered to the colon, leading to a higher bioavailability if drug absorption through the colonic mucosa is favorable.

The outer enteric coating disintegrates in the intestine, preferably in the terminal ileum, most preferably in the colon of the patient, releasing the individual core units covered with the mucoadhesive material. Using individual core units in the dosage form of the present invention rather than a single unit form provides better dispersion and distribution along the intestinal wall, preferably the colonic mucosa, i.e. the individual units are widely spread with the result that local high dose concentration, dose dumping and irritation compared to single unit dosage forms is diminished and preferably avoided. Furthermore, individual units have a slower transit time compared to single unit dosage forms, allowing an extended time for complete drug release.

Providing the individual units with a mucoadhesive material has the advantage that the adhesion to the intestinal wall (mucus layer), preferably the colonic mucosa is improved so that the individual units are retained in the intestine, preferably in the colon, for a prolonged time due to adhesive interaction with the intestine wall, preferably with the colonic mucus layer. The retention of the particles at the mucosal layer increases the residence time of the particles in the target region, thereby improving bioavailability and, in consequence, therapeutic efficacy and reproducibility of the therapeutic efficacy, especially in patients with increased peristalsis of the digestive system and chronic inflammation of the intestine.

The term "dosage form", as used herein, refers to the formulation of a pharmaceutically active ingredient, which is suitable for (preferably orally) administering said pharmaceutically active ingredient to a patient, such as e.g. a tablet or a capsule.

The term "multiple unit dosage form", as used herein, means that the dosage form comprises a plurality of small(er) sub-units such as pellets, granules, which comprise the pharmaceutically active ingredient. It is characteristic for a multiple unit dosage form that the individual units are dispersed and distributed in the target region of the patient upon administration of the dosage form. A multiple unit dosage form is thus different from a single unit dosage form which only comprises one unit.

The term "pharmaceutically active ingredient", as used herein, refers to an ingredient of the multiple unit dosage form which provides a therapeutic effect, in particular in order to treat or prevent a disease of a patient. Thus, a pharmaceutically active ingredient is not a pharmaceutically acceptable excipient. The "pharmaceutically active ingredient" can also be designated "as pharmaceutically active agent".

The term "mucoadhesion" is commonly defined as the adhesion between two materials, at least one of which is a mucosal layer. In the context of the present invention, the term "mucoadhesive" refers to the property of a material comprised in the multiple unit dosage form to adhesively interact with the colonic mucosa (gastrointestinal mucus). Thus, the term "mucoadhesive material", as used herein, refers to a material which is able to adhesively interact with the colonic mucosa (gastrointestinal mucus).

The term "enteric coating layer", as used herein, refers to an outer coating layer of the dosage form, which is substantially insoluble in the stomach of a patient, and soluble in the intestine of a patient, i.e. which does not disintegrate in the stomach but disintegrates in the intestine, preferably close to or in the colon. In consequence, the dosage form is substantially insoluble in the stomach of a patient, and the active ingredient is substantially, preferably completely, not released in the stomach of the patient, but in the intestine, preferably in the colon.

By "substantially insoluble", it is understood that 1 g of the material requires more than 10,000 ml of solvent (surrounding medium) to dissolve at a given pH. By "soluble", it is understood that 1 g of the material requires less than 10000 ml, preferably less than 5000 ml, more preferably less than 1000 ml, even more preferably less than 100 ml or 10 ml of solvent to dissolve at a given pH. "Surrounding medium" means the medium in the gastrointestinal tract, such as the gastric fluid or intestinal fluid. Alternatively, the surrounding medium may be an in vitro equivalent of the medium in the gastrointestinal tract.

Testing whether a dosage form comprising an outer enteric coating layer is substantially insoluble in the stomach and soluble in the intestine can e.g. be done using the USP Dissolution Apparatus 2 (Paddle). First, the dosage form is placed in 900 mL 0.1 N hydrochloric acid, stirred at 50 rpm at a temperature of 37±0.5 °C for 2 hours. An aliquot of the solution is taken. Next, the dosage form is placed in Hanks buffer (pH 6.8, composition disclosed in Example 3) or Krebs buffer (pH 7.4, composition disclosed in Example 3), stirred at 50 rpm at a temperature of 37±0.5 °C for 60 minutes. An aliquot of the solution is taken. Then, the amount of the active ingredient in both aliquots is measured in a suitable assay. With respect to additional details of the process it is referred to the US or European Pharmacopeia.

The abbreviation "wt.-%", as used herein, means "weight percentage", and refers to the partial weight amount in relation to a total (overall) weight amount, as specifically indicated under the circumstances. If nothing else is indicated or obvious under the circumstances, "w/w" means "wt.-%".

The term "comprising", as used herein, includes "consisting essentially of" and "consisting of". The term "molecular weight" or "Mw" of a polymer, as used herein, refers to the weight average molecular weight of the corresponding polymer, as determined by GPC if nothing else is explicitly stated or obvious under the circumstances.

The use of the term "about", in particular in conjunction with the molecular weight (Mw) of the polymer, attempts to address the fact that the molecular weight of a polymer cannot exactly be determined and underlies measurement fluctuations. For the purpose of the present invention, the term "about" used in conjunction with the molecular weight (Mw) of a polymer thus refers the molecular weight (Mw) of the polymer ± 20%, preferably ± 10%. The term "about" is furthermore used in connection with the pH value and addresses the fact the pH value might also be subject to slight variations when being measured.

The term "patient" as used herein, refers to a mammal patient or a human patient, preferably to a human patient.

### The outer coating layer (b1) of the core coating (b)

The core (a) of the multiple unit dosage form of the present invention is completely covered by a core coating (b). Said core coating (b) comprises an outer coating layer (b1), which is an enteric coating layer being substantially insoluble in the stomach of a patient and soluble in the intestine of a patient.

This outer enteric coating layer (b1) of the core coating (b) allows the multiple unit dosage form of the present invention to pass the stomach passage of a patient, which usually has a pH value of about 1 to about 3 in a manner that substantially none (not more than 10%, preferably not more than 5%, more preferably not more than 2%), preferably none of the pharmaceutically active ingredient is released from the dosage form in the stomach passage.

In a preferred embodiment of the present invention, the outer coating layer (b1) of the core coating (b) is an enteric coating layer which comprises a polymeric material with a pH threshold of about pH 5 or above, i.e. which dissolves at a pH threshold of about pH 5 or above and is substantially insoluble at more acidic pH values such as the pH value in the stomach passage of a patient.

The polymeric material is thus pH sensitive and dissolves in a pH dependent manner, i.e. has a "pH threshold" which is the pH below which it is insoluble in aqueous media and at or above which it is soluble in aqueous media. Thus, the pH of the surrounding medium triggers dissolution of the polymeric material and none (or substantially none) of the polymeric material dissolves below the pH threshold. Once the pH of the surrounding medium reaches (or exceeds) the pH threshold, the polymeric material becomes soluble. By "surrounding medium", the gastric fluid and intestinal fluid, or an aqueous solution designed to simulate in vitro gastric fluid or intestinal fluid is meant. The normal pH of gastric fluid is usually in the range of about pH 1 to about pH 3. The polymeric material is insoluble below pH 5 and soluble at about pH 5 or above and, thus, is usually insoluble in gastric fluid. Such a material may be referred to as a gastro-resistant material or, as used above, an "enteric" material.

The polymeric material has a pH threshold of pH 5 or above, e.g. about pH 5.5 or above, preferably about pH 6 or above and more preferably about pH 6.5 or above. The polymeric material typically has a pH threshold of no more than about pH 8, e.g. no more than about pH 7.5 and preferably no more than about pH 7.2. Preferably, the polymeric material has a pH threshold within the range of pH found in intestinal fluid. The pH of intestinal fluid may vary from one person to the next, but in healthy humans is generally from about pH 5 to 6 in the duodenum, from about 6 to 8 in the jejunum, from about 7 to 8 in the ileum, and from about 6 to 8 in the colon. The polymeric material preferably has a pH threshold of about 6.5, i.e. is insoluble below pH 6.5 and soluble at about pH 6.5 or above, and more preferably has a pH threshold of about 7, i.e. is insoluble below pH 7 and soluble at about pH 7 or above. The pH threshold at which a material becomes soluble may be determined by a simple titration technique which would be part of the common general knowledge to the person skilled in the art.

The polymeric material is typically a film-forming polymeric material such as a polymethacrylate polymer, a cellulose polymer or a polyvinyl-based polymer.

Examples of suitable cellulose polymers include cellulose acetate phthalate (CAP); cellulose acetate trimellitate (CAT); hydroxypropylmethylcellulose phthalate and hydroxypropylmethylcellulose acetate succinate (HPMC-AS).

Examples of suitable polyvinyl-based polymers include polyvinyl acetate phthalate (PVAP).

Preferably, the polymeric material is a polymethacrylate polymer, more preferably an "anionic" polymethacrylate polymer which contains groups that are ionizable in aqueous media to form anions.

Preferably, the polymethacrylate polymer is a co-polymer of a (meth)acrylic acid and a (meth)acrylic acid C1-4 alkyl ester, for example, a co-polymer of methacrylic acid and methacrylic acid methyl ester. Such a polymer is known as a poly(methacrylic acid/methyl methacrylate) co-polymer. Suitable examples of such co-polymers are usually anionic and not sustained release polymethacrylates. The ratio of carboxylic acid groups to methyl ester groups (the "acid:ester ratio") in these co-polymers determines the pH at which the co-polymer is soluble. The acid:ester ratio may be from about 2:1 to about 1:3, e.g. about 1:1 or, preferably, about 1:2. The molecular weight of preferred anionic co-polymers is usually from about 120000 to 150000 g/mol, preferably about 125000 g/mol or about 135000 g/mol.

Preferred anionic poly(methacrylic acid/methyl methacrylate) co-polymers have a molecular weight of about 125000 g/mol. Suitable examples of such polymers have an acid:ester ratio of about 1:1 and a pH threshold of about pH 6, or have an acid:ester ratio of about 1:2 and a pH threshold of about pH 7.

A specific example of a suitable anionic poly(methacrylic acid/methyl methacrylate) co-polymer having a molecular weight of about 125000 g/mol, an acid:ester ratio of about 1:1 and a pH threshold of about pH 6 is sold under the trade mark Eudragit® L. This polymer is available in the form of a powder (Eudragit® L 100), or as an organic solution (12.5%) (Eudragit® L 12.5).

A specific example of a suitable anionic poly(methacrylic acid/methyl methacrylate) co-polymer having a molecular weight of about 125000 g/mol, an acid:ester ratio of about 1:2 and a pH threshold of about pH 7 is sold under the trade mark Eudragit® S. This polymer is available in the form of a powder (Eudragit® S 100) or as an organic solution (12.5%) (Eudragit® S 12.5).

The polymeric material may be a co-polymer of methacrylic acid and ethyl acrylate. Preferred poly(methacrylic acid/ethyl acrylate) co-polymers have a molecular weight from about 300000 to 350000 g/mol, e.g. about 320000 g/mol. Suitable examples of such co-polymers have an acid:ester ratio of about 1:1 and a pH threshold of about pH 5.5.

A specific example of a suitable anionic poly(methacrylic acid/ethyl acrylate) co-polymer is available in the form of a powder and sold under the trade mark Eudragit® L 100-55, or in the form of an aqueous dispersion (30%) and sold under the trade mark Eudragit® L 30 D-55.

The polymeric material may be a co-polymer of methyl acrylate, methyl methacrylate and methacrylic acid. Preferred poly(methyl acrylate/methyl methacrylate/methacrylic acid) co-polymers have a molecular weight from about 250000 to about 300000 g/mol, e.g. about 280000 g/mol. Suitable examples of such co-polymers have a methyl acrylate:methyl methacrylate:methacrylic acid ratio of 7:3:1 thereby providing an acid:ester ratio of 1:10 and a pH threshold of about pH 7.

A specific example of a suitable anionic poly(methyl acrylate/methyl methacrylate/ethyl acrylate) co-polymer is available in the form of an aqueous dispersion (30%) and is sold under the trademark Eudragit® FS 30 D.

The Eudragit® co-polymers are manufactured and/or distributed by Evonik GmbH, Darmstadt, Germany.

Mixtures of film forming polymer materials may be used as appropriate. For example, the polymeric material may be a blend of at least two different polymers having a pH threshold of about pH 5 and above. Preferably, the polymers in the blend are different polymethacrylate polymers. In embodiments where the polymeric material is a blend of two different polymers having a pH threshold of about pH 5 or above, the polymers may be present in the blend in a polymer weight ratio from 1:99 to 99:1, e.g. from 10:90 to 90:10, or from 25:75 to 75:25, or from 40:60 to 60:40, for example 50:50.

In a further preferred embodiment of the present invention, the outer coating layer (b1) comprises in addition or alternatively to the above described polymeric material having a pH threshold of about pH 5 or above a polymeric material which is susceptible to an attack by colonic bacteria.

Said polymeric material susceptible to an attack by colonic bacteria typically comprises a polysaccharide, preferably containing a plurality of glucose units, e.g. a polyglucoside. In a preferred embodiment, the polysaccharide is at least one polysaccharide selected from the group consisting of starch, amylose, amylopectin, chitosan, chondroitin sulfate, cyclodextrin, dextran, pullulan, carrageenan, scleroglucan, chitin, curdulan, levan, guar gum and alginate. It is further preferred that the polysaccharide is starch, amylose or amylopectin, most preferably starch.

The person skilled in the art is capable of determining whether a polymeric material is susceptible to attack by colonic bacteria using techniques comprising part of the common general knowledge. For example, a pre-determined amount of a given material could be exposed to an assay containing an enzyme from a bacterium found in the colon and the change in weight of the material over time may be measured.

As noted above, the polysaccharide is preferably starch. Starches are usually extracted from natural sources such as cereals, pulses, and tubers. Suitable starches for use in the present invention are typically food grade starches and include rice starch, wheat starch, corn (or maize) starch, pea starch, potato starch, sweet potato starch, tapioca starch, sorghum starch, sago starch, and arrow root starch.

Starch is typically a mixture of two different polysaccharides, namely amylose and amylopectin. Different starches may have different proportions of these two polysaccharides.

Starches suitable for use in the present invention typically have at least 0.1 wt.-%, preferably at least 10 wt.-%, more preferably at least 35 wt.-% amylose, based on the total weight of the starch. Preferred starches have no more than 50 wt.-% amylopectin, preferably no more than 40 wt.-% amylopectin, based on the total weight of the starch. A method for determining the relative proportions of amylose and amylopectin in starch is described e.g. in WO2013/164315.

Preferred starches are "off-the-shelf' starches, i.e. starches which require no processing prior to use in the context of the present invention. Examples of particularly suitable "high amylose" starches include Hylon™ VII (National Starch, Germany), Eurylon™ 6 (or VI) or Amylo NI-460 or Amylo N-400 (Roquette, Lestrem, France), or Amylogel 03003 (Cargill, Minneapolis, USA) all of which are examples of a maize starch having from 50 wt.-% to 75 wt% amylose.

In a preferred embodiment of the present invention, the outer coating layer (b1) comprises a mixture of the polymeric material with a pH threshold of about 5 or above ("pH trigger") and the polymeric material which is susceptible to an attack by colonic bacteria ("bacteria trigger"). The proportion of the bacteria trigger to the pH trigger in said mixture is typically at least 1:99, e.g. at least 10:90 and preferably at least 25:75. The proportion is typically no more than 99:1, e.g. no more than 75:25 and preferably no more than 60:40. In some embodiments, the proportion may be no more than 35:65. In some preferred embodiments, the proportion is from 10:90 to 75:25, e.g. from 10:90 to 60:40 and preferably from 25:75 to 60:40. In some particularly preferred embodiments, the proportion is from 15:85 to 35:65, e.g. from 25:75 to 35:65 and preferably 30:70. In other particularly preferred embodiments, the proportion is from 40:60 to 60:40, e.g. 50:50.

In a particularly preferred embodiment of the present invention, the outer coating layer (b1) comprises an anionic co-polymer of a (meth)acrylic acid and a (meth)acrylic acid C1-4 alkyl ester, wherein the ratio of methacrylic acid to methacrylic acid methyl ester is preferably 1:2, as the pH trigger, and starch, preferably comprising at least 35 wt.-% amylose, based on the total weight of the starch, as the bacteria trigger, the proportion of the bacteria trigger and the pH trigger preferably being 30:70 or 50:50.

The thickness of the outer coating layer (b1) of the core (b) is typically from 10 µm to 150 µm. The thickness of a specific coating will, however, depend on the composition of the coating. For example, coating thickness is directly proportional to the amount of polysaccharide in the coating and the total coating composition. Thus, in embodiments where the coating comprises high amylose starch and Eudragit® S at a ratio of 30:70, the coating thickness may be from 70 µm to 130 µm, and preferably from 90 µm to 110 µm. The thickness (in µm) for a given coating composition is independent of core size.

The thickness of a layer or coating on an oral dosage form such as a tablet, is generally measured by subjecting the cross section of the dosage form to scanning electron microscopy (SEM) and then by using the measurement software of the SEM instrument (i.e. Phenom SEM measurement software) or any other measurement software like MeasurelT from Olympus Soft Imaging Solutions GmbH. However, SEM may not be specific enough in some cases, including in cases where adjacent layers cannot be distinguished properly, or where the typical margin of error in SEM (about 5 to 10%) is not acceptable. In such cases, the thickness of the coating or layer to be distinguished can be determined precisely using atomic force microscopy (AFM) or terahertz pulsed spectroscopy and imaging (TPI). A method of using TPI to measure the thickness of a layer in a tablet is described in the Journal of Pharmacy and Pharmacology (2007), 59: 209-223.

### Further coating layer (b2) of the coating (b)

In a preferred embodiment of the present invention, the core coating (b) of the multiple unit dosage form of the present invention comprises a further coating layer (b2), which is between the core (a) and the outer coating layer (b1).

Preferably, the further coating (b2) is only present in core coating (b) of the multiple unit dosage form of the present invention if the outer enteric layer (b1) does not comprise a polymeric material which is susceptible to an attack by colonic bacteria ("bacteria trigger"). If the outer enteric layer (b1) comprises a polymeric material which is susceptible to an attack by colonic bacteria ("bacteria trigger"), the further coating (b2) is preferably not present.

The further coating layer (b2) comprises a polymeric material which is soluble in intestinal fluid or gastric fluid. The further coating layer (b2) facilitates disintegration of the outer coating layer (b1) in the intestine, preferably in the colon, by forming a fluid region between the core and the outer coating layer. Thereby, it contributes to the release and the distribution of the individual units covered with a mucoadhesive material and further comprising the active ingredient in the intestine, preferably in the colon of a patient.

By "intestinal fluid", the fluid in the lumen of the intestine of a mammal, particularly a human, is meant. Intestinal fluid is a pale yellow aqueous fluid secreted from glands lining the walls of the intestine. Intestinal fluid includes fluid found in the small intestine, i.e. fluid found in the duodenum (or "duodenal fluid"), fluid found in the jejunum (or "jejunal fluid") and fluid found in the ileum (or "ileal fluid"), and fluid found in the large intestine, e.g. "colonic fluid".

By "gastric fluid", the aqueous fluid in the stomach of a mammal, particularly a human is meant. The fluid contains up to about 0.1 N hydrochloric acid and substantial quantities of potassium chloride and sodium chloride, and plays a key role in digestion by activating digestive enzymes and denaturing ingested protein. Gastric acid is produced by cells lining the stomach and other cells produce bicarbonate which acts as a buffer to prevent the gastric fluid from becoming too acidic.

The skilled person can readily determine whether a polymer is soluble in intestinal or gastric fluid. If a polymer is soluble in water (or aqueous solution, e.g. a buffer solution) at a pH from 5 to 8, then that polymer would typically be soluble in intestinal fluid. Alternatively, the compositions of intestinal fluid are known and may be replicated in vitro. If a polymer is soluble in artificial intestinal fluid in vitro, then it would typically be soluble in intestinal fluid respectively in vivo.

Any pharmaceutically acceptable water soluble film forming polymers are, in principle, suitable for use as the polymeric material for the further coating layer (b2).

In a preferred embodiment, the polymeric material is selected from the group consisting of a polycarboxylic acid polymer that is at least partially neutralized and a non-ionic polymer, each in combination with at least one additive selected from a buffer agent and/or a base and mixtures thereof.

Suitable non-ionic polymers for use as the polymeric material for the further coating layer (b2) include pharmaceutically acceptable polymers which do not ionize in aqueous media. Examples of suitable non-ionic polymers include methylcellulose (MC), hydroxypropyl cellulose (HPC), hydroxypropyl methylcellulose (HPMC), poly(ethyleneoxide)-graft-polyvinylalcohol, polyvinylpyrrolidone (PVP), polyethylene glycol (PEG) and polyvinylalcohol (PVA).

The partially neutralized polycarboxylic acid polymer for use in the further coating layer (b2) comprises at least a portion, e.g. at least 10 %, preferably at least 25 %, more preferably at least 50 %, and most preferably at least 90 %, of the carboxylic acid groups in form of carboxylate anions. In particularly preferred embodiments, all of the carboxylic acid groups in the polymeric material are in the form of carboxylate anions. Such polymers are referred to herein as "fully neutralized".

Examples of suitable polycarboxylic acid polymers include polymethacrylates, cellulose acetate phthalate (CAP), polyvinyl acetate phthalate (PVAP), hydroxypropyl methylcellulose phthalate (HPMCP), hydroxypropyl methylcellulose acetate succinate (HPMC-AS), cellulose acetate trimellitate (CAT), xanthan gum, alginates and shellac.

Preferably, however, the polycarboxylic acid polymer is selected from co-polymers of a (meth)acrylic acid and a (meth)acrylic acid alkyl, e.g. C1-4 alkyl, ester and a co-polymer of methacrylic acid and methacrylic acid methyl ester is particularly suitable. Such a polymer is known as a poly(methacrylic acid/methyl methacrylate) co-polymer or a "polymethacrylate".

The ratio of carboxylic acid groups to methyl ester groups (the "acid:ester ratio") in these co-polymers determines the pH at which the co-polymer is soluble. The acid:ester ratio may be from 2:1 to 1:3, e.g. 1:1 or, preferably, 1:2. The molecular weight of preferred co-polymers is usually from about 120000 to 150000, preferably about 125000 or about 135000.

Preferred polymeric materials for the further coating layer (b2) include e.g. Eutragit® L; Eutragit® S, Eutragit® FS 30 D, Eutragit® L30D-55, and Eutragit L100-55, as discussed above. These are used in at least partially, more preferably fully neutralized form.

In preferred embodiments, the polymeric material of the further coating layer (b2) is an at least partially, preferably fully neutralized co-polymer of (meth)acrylic acid and a (meth)acrylic acid C1-4 alkyl ester. In particularly preferred embodiments, the polymeric material of the further coating layer (b2) is a fully neutralized co-polymer of (meth)acrylic acid and (meth)acrylic acid methyl ester, particularly Eudragit® S.

Fully neutralized Eudragit® S is capable of forming a film(optionally in the presence of a plasticizer) and is readily and completely soluble in water independently of at least the range of pH found in the intestine, e.g. about pH 5 to about pH 8. Fully neutralized Eudragit® S is particularly preferred for use as the polymeric material of the further coating layer (b2) in the present invention.

The further coating layer (b2) of these embodiments preferably comprises a base and/or a buffer agent. In preferred embodiments, the further coating layer (b2) comprises both a buffer agent and a base.

The purpose of the base is to provide an alkaline environment on the underside of the outer layer once intestinal fluid begins to penetrate the outer layer. In principle, any pharmaceutically acceptable base may be used. The base is typically a non-polymeric compound. Suitable bases include inorganic bases such as sodium hydroxide, potassium hydroxide and ammonium hydroxide, and organic bases such as triethanolamine, sodium bicarbonate, potassium carbonate, trisodium phosphate, trisodium citrate or physiologically tolerated amines such as triethylamine. Hydroxide bases in general, and sodium hydroxide in particular, are preferred.

In view of the above, it is preferred that the further coating layer (b2) of the core coating (b) is an alkaline layer, which means that the layer material when dissolved in 10 times its weight of water provides a pH value above the pH at which the enteric polymer in the outer coating b1 dissolves.

The further coating layer (b2) preferably comprises at least one buffer agent. The purpose of the buffer agent is to provide or increase pH buffer capacity on the underside of the outer layer once intestinal fluid begins to penetrate the outer layer. The buffer agent may be an organic acid such as a pharmaceutically acceptable non-polymeric carboxylic acid, e.g. a carboxylic acid having from 1 to 16, preferably 1 to 3, carbon atoms. Suitable carboxylic acids are disclosed in WO2008/135090A. Citric acid is an example of such a carboxylic acid. The carboxylic acids may be used in carboxylate salt form, and mixtures of carboxylic acids, carboxylate salts or both may also be used.

The buffer agent may also be a pharmaceutically acceptable inorganic salt such as an alkali metal salt, an alkali earth metal salt, an ammonium salt, and a soluble metal salt. As metals for the soluble metal salts, manganese, iron, copper, zinc and molybdenum can be mentioned. Further preferred, the pharmaceutically acceptable inorganic salt is selected from chloride, fluoride, bromide, iodide, phosphate, nitrate, nitrite, sulphate and borate. Phosphates such as potassium dihydrogen phosphate are preferred over other inorganic buffer salts and organic acid buffers due to their greater buffer capacity at the pH of 8.

The buffer is usually present in the further coating layer (b2) in an amount from 0.1 to about 20 wt.-%, e.g. from 0.1 to 10 wt.-%, preferably from 0.1 to 4 wt.-%, even preferably from 0.1 to 3 wt.-%, and even more preferably 1 wt.-%, based on the dry weight of the polymeric material of the further coating layer (b2). This particularly applies for cases where polymers which are at least partially neutralized are present because most of the buffer capacity is given by the neutralized polymer. For non-ionic polymers, the buffer capacity is given by the buffer salt, therefore, higher concentrations are required to reach a high buffer capacity to accelerate dissolution of the outer coating. In this case, the buffer can be present in an amount of up to about 50%.

The thickness of the further coating layer (b2) of the core (b) is typically from 10 µm to 150 µm.

### Isolation layer (c)

The multiple unit dosage form of the present invention can optionally further comprise an isolation layer (c) between the core (a) and the core coating (b). The use of an isolation layer (c) between the core (a) and the core coating (b) further contributes to the disintegration of the enteric coating layer (b1) in the intestine, preferably in the colon of the patient, and accelerates release of the individual units comprising a mucoadhesive material and comprising the active ingredient.

The isolation layer (c) typically comprises at least one non-ionic polymer. Suitable polymers include at least one polymer selected from the group consisting of methylcellulose (MC); hydroxypropyl cellulose (HPC); hydroxypropyl methylcellulose (HPMC); poly(ethylene oxide)-graft-polyvinyl alcohol; polyvinylpyrrolidone (PVP); and polyvinyl alcohol (PVA).

In preferred embodiments, the isolation layer (c) comprises HPMC. In other preferred embodiments, the isolation layer (c) comprises PVA.

The non-ionic polymer is typically present in the isolation layer (c) as the sole film-forming polymeric material.

The polymeric material of the isolation layer (c) is preferably present in the isolation layer (c) in a total amount from 1 mg polymer/cm² to 5 mg polymer/cm², preferably from 2 mg polymer/cm² to 4 mg polymer/cm², more preferably from 2.5 mg polymer/cm² to 3.5 mg polymer/cm², and most preferably of 3 mg polymer/cm².

The thickness of the isolation layer (c) is typically from 5 µm to 100 µm, preferably from 10 µm to 60 µm, and most preferably from 20 µm to 40 µm.

In a preferred embodiment of the present invention, the multiple unit dosage form of the present invention comprises an isolation layer (c) between the core (a) and the core coating (b), wherein the core coating (b) comprises an outer coating layer (b1) which comprises a mixture of the polymeric material with a pH threshold of about 5 or above ("pH trigger") and the polymeric material which is susceptible to an attack by colonic bacteria ("bacteria trigger"). In a particularly preferred embodiment of the present invention, the multiple unit dosage form of the present invention comprises an isolation layer (c) between the core (a) and the core coating (b), wherein the isolation layer (c) comprises HPMC or PVA; and wherein the core coating (b) comprises an outer coating layer (b1) which comprises an anionic co-polymer of a (meth)acrylic acid and a (meth)acrylic acid C1-4 alkyl ester, wherein the ratio of methacrylic acid to methacrylic acid methyl ester is preferably 1:2, as the pH trigger, and starch, preferably comprising at least 35 wt.-% amylose, based on the total weight of the starch, as the bacteria trigger, the proportion of the bacteria trigger and the pH trigger preferably being 30:70 or 50:50.

### The core (a), the at least one pharmaceutically active ingredient (a1) and the at least one mucoadhesive material (a2)

The multiple unit dosage of the present invention comprises a core (a). The core (a) can e.g. represent a tablet or a hard- or soft-shell capsule, a pellet, a micro-tablet or granules. Preferably, the core represents a tablet or a hard-shell capsule (either gelatin or HPMC hard shell capsule), especially preferably a hard-shell capsule.

The core (a) of the multiple unit dosage form of the present invention comprises a plurality of individual core units, each unit covered by at least one mucoadhesive material (a2). The individual core units can also be coated with one or more coating layers with the mucoadhesive material (a2) covering the individual core units comprising one or more coating layers.

### "Covering" includes complete covering but also partial covering

The at least one mucoadhesive material (a2) covering the individual units can be included in a mucoadhesive coating layer or in a mucoadhesive matrix, wherein said layer/matrix covers the individual units.

For example, the individual core units can be coated with one or more coating layers, wherein the outer coating layer represents a mucoadhesive coating layer comprising the at least one mucoadhesive material (a2). Alternatively, the individual core units, optionally including one or more coating layers, can be included in a mucoadhesive matrix, comprising the at least one mucoadhesive material (a2). Preferably, the at least one mucoadhesive material (a2) is included in a mucoadhesive coating layer covering the individual core units forming the core (a).

In the embodiments in which the individual core units are embedded in a mucoadhesive matrix, this mucoadhesive matrix breaks up once the core coating (b) is removed, and each individual core unit then has some mucoadhesive material attached, which can "glue" the individual core unit to the mucosa.

The use of individual units covered by a mucoadhesive material (a2) and forming the core (a) of the multiple unit dosage form is favorable in terms of the mucoadhesivity compared to single unit formulations due to better dispersion and distribution along the intestinal lumen, preferably in the colonic lumen, and the increased total surface area available for adhesive bonds with the mucus layer. Also, the gastrointestinal transit of individual units as described herein is less variable and transit through the colon is slower compared to single unit formulations due to a sieving effect. Thus, a dosage form combining mucoadhesive features in multiple individual units contributes to an overall increased gastrointestinal residence time.

Preferably, the individual units are selected from microparticles, granules, pellets, and mini tablets, with pellets being more preferred as individual core units. The individual units can be porous or non-porous drug carriers, preferably they are porous. The individual units can alternatively also be crystals of the active ingredient.

Porous individual units are capable of associating active ingredients. The association can be an adsorption onto the surface of the particles, be it the outer or the inner surface of the particles or an absorption into the particles. Adsorption and absorption of the active ingredient is essentially controlled by the pore size, which preferably is in the range of from 10 to 100 nm, more preferably in the range of between 20 and 80 nm, especially from 30 to 70 nm, such as 50 nm.

Preferably, each individual unit has a maximum outer diameter in the range of 1 µm, preferably, 10 µm, more preferably 100 µm to 1 mm, even more preferably in the range of 100 µm to 500 µm. The particle size of the individual units is important with respect to mucoadhesion and manufacturability. Smaller particles have better mucoadhesivity due to the increased surface area-to-volume ratio. However, compared to nanoparticles, microparticles have the advantage of an easier manufacturability due to improved flowability. Size tuning of the particles in the micrometer range furthermore offers the possibility to specifically target inflamed regions in the gastrointestinal tract.

Each individual unit of the core (a) comprises at least one pharmaceutically active ingredient (a1). The pharmaceutically active ingredient is not particularly limited and can be selected by the skilled person according to the needs. Preferably, the pharmaceutically active ingredient is an anti-inflammatory agent or antibiotic, and e.g. selected from metronidazole, vancomycine, 5-amino-salicylic acid, budesonide, and anti-TNFs. For example, a patient suffering from inflammatory bowel disease, especially if having moderate to severe disease is typically also being treated with mesalazine or derivatives or prodrugs thereof, corticosteroids, e.g. budesonide or prednisolone (oral or i.v.), immunosuppressants, e.g. azathioprine/6-mercaptopurine (6-MP) or methotrexate, cyclosporine or tacrolimus. Other active ingredients which can be co-administered to the patient include biologics such as infliximab, adalimumab, etanercept, certolizumab pegol or others. Further active ingredients which can be co-administered to the patient include immunosupressants (e.g. azathioprine/6-MP or methotrexate or oral cyclosporine) in order to maintain stable and longer remission. Yet another aspect of the invention is the use of an anti-TNFα antibody or functional fragment as defined hereinabove for reducing inflammation.

The pharmaceutically active ingredient can be present in its free base or acid form or as any pharmaceutically acceptable derivative such as ester and/or salt thereof.

The pharmaceutically active ingredient can e.g. be incorporated into the individual units by means of absorption or it can e.g. be applied by means of a coating comprising the pharmaceutically active ingredient.

Preferably, the pharmaceutically active ingredient is present in the individual units in an amount of at least 10 wt.-%, more preferably at least 20 wt.-%, even more preferably at least 30 wt.-%, and even more preferably at least 40 wt.-%, even more preferably at least 50%, based on the total weight of the individual unit comprising the pharmaceutically active ingredient (without the mucoadhesive material (a2)). The drug loading of the individual units can also be 90 to 100 wt.-%, e.g. if pellets obtained by extrusion-spheronization or drug crystals are used as individual units.

Covering the individual units by at least one mucoadhesive material (a2) has the advantage that the individual units are retained in the intestine for a prolonged time so that the active ingredient (a1) may be released also for a prolonged time without the risk that the carrier particles leave the intestine before the active agent has been sufficiently released. The retention of the particles at the mucosal layer extends the residence time in the target region.

Suitable mucoadhesive materials are known to a person skilled in the art, and include synthetic, natural, and semi-synthetic mucoadhesive materials.

Examples for synthetic mucoadhesive polymers include various acrylic acid derivatives, in particular carbopol, polycarbophil, polyacrylic acid, polyacrylates, poly(methylvinylether-co-methacrylic) acid, poly(2-hydroxyethyl methacrylate), poly(methacrylate), poly(alkylcyanoacrylate), poly(isohexylcyanoacrylate), poly(isobutylcyanoacrylate), polyacrylates (also known as carbomers which are commercially available as Carbopols®). Further synthetic mucoadhesive polymers are polyvinylpyrrolidone (PVP) and polyvinylalcohol. Locust bean gum, alginate, pectin, xanthan gum, carrageenan, guar gum, hyaluronate, tamarind gum, and chondroitin sulphate can be mentioned as examples for natural mucoadhesive polymers. Chitosan and many cellulose derivatives such as carboxymethyl cellulose, hydroxyethyl cellulose, hydroxypropyl cellulose, sodium carboxymethyl cellulose, methyl cellulose, methylhydroxyethyl cellulose, and hydroxypropylmethyl cellulose are known as semi-synthetic mucoadhesive polymers.

In a preferred embodiment of the present invention, the at least one mucoadhesive material (a2) comprises chitosan or alginate, more preferably chitosan.

In an especially preferred embodiment of the multiple unit dosage form of the present invention, the at least one mucoadhesive material (a2) comprises chitosan; the core coating (b) comprises an outer coating layer (b1), comprising a mixture of an anionic co-polymer of a (meth)acrylic acid and a (meth)acrylic acid C1-4 alkyl ester, wherein the ratio of methacrylic acid to methacrylic acid methyl ester is preferably 1:2, as the pH trigger, and starch, preferably comprising at least 35 wt.-% amylose, based on the total weight of the starch, as the bacteria trigger, the proportion of the bacteria trigger and the pH trigger preferably being 30:70 or 50:50. It is even more preferred to include an isolation layer (c) between the core (a) and the core coating (b), said isolation layer (c) preferably comprising HPMC or PVA.

Preferably, each individual core unit releases the active ingredient over an extended time period. This can be achieved e.g. by coating each individual core unit with a controlled release coating or by providing each individual core unit as a controlled release matrix, as is known in the art. If the mucoadhesive material (a2) is coated as a layer on each individual core unit, this layer can also contain a controlled release polymer so that a layer is formed, which is both mucoadhesive and provides controlled release of the active ingredient (a1). If the individual core units are embedded in a mucoadhesive matrix, it is preferred that each individual core unit comprises a controlled release matrix.

The active ingredient (a1) is released from the controlled release coating layer or the controlled release matrix over a time period of at least 30 minutes, preferably at least 60 minutes, more preferably at least 120 minutes, even more preferably at least 240 minutes, even more preferably at least 480 minutes, even more preferably at least 720 minutes when tested under the following conditions:
*In vitro* dissolution studies are performed on a USP type II apparatus using a paddle speed of 50 rpm and a media temperature of 37 ± 0.5 °C. Capsules or tablets are first treated in 0.1 M HCl for 2 h, followed by treatment in Krebs buffer (pH 7.4), which closely resembles the electrolyte composition of the human ileo-colonic fluid. The pH of the buffer is stabilized at 7.4 ± 0.05 by continuously sparging with 5% CO₂ / 95% O₂. Absorbance measurements are taken at regular intervals, the composition per liter of Krebs buffer is 0.16 g of KH₂PO₄, 6.9 g of NaCl, 0.35 g KCI, 0.29 g MgSO₄·7H₂O, 0.376 g CaCl₂·2H₂O and 2.1 g NaHCO₃. The time period of the release starts when the sample is treated with the Krebs buffer.

The mucoadhesive coating layer or the mucoadhesive matrix comprising the mucoadhesive material (a2) and covering the individual core units can further comprise an amine methylacrylate co-polymer or ethyl cellulose or a mixture thereof.

In a further preferred embodiment of the present invention, the individual units of core (a) comprise an inert seed core onto which a first coating layer is coated containing the pharmaceutically active ingredient (a1) which is covered with the mucoadhesive material (a2) in form of a mucoadhesive coating layer or embedded in a mucoadhesive matrix. The term "inert" means that the seed does not chemically react with the pharmaceutically active agent during the preparation and storage of the multiple dosage form.

In another preferred embodiment of the present invention, the seed core comprises, preferably consists of functionalized calcium carbonate (FCC - Omyapharm) as porous drug carrier.

Functionalized calcium carbonate is known in the art. It is a highly porous variation of precipitated calcium carbonate. As a result of its small pore size and enlarged surface area it is called "functionalized calcium carbonate" (FCC). The particle size and porosity can be tailored according to customer demands.

FCC can be prepared as described in WO00/39222 and EP-A-2 264 108. Since it is prepared by reaction of the surface of natural or synthetic calcium carbonate with carbon dioxide and an acid, FCC is also called "surface-reacted calcium carbonate". Thus, FCC is obtainable by reacting natural or synthetic calcium carbonate with carbon dioxide and one or more acids, wherein the carbon dioxide is formed in situ by the acid treatment and/or is supplied from an external source as described for example in WO2010/037753.

The porous individual units preferably have a specific surface area of from 5 m²/g to 200 m²/g, more preferably from 20 m²/g to 80 m²/g and most preferably from 30 m²/g to 60 m²/g, measured using nitrogen and the BET method according to ISO9277.

The porous individual units preferably have a weight median grain diameter d₅₀ of from 0.1 to 50 µm, more preferably from 0.5 to 25 µm, even more preferably from 0.8 to 20 µm, particularly from 1 to 10 µm measured according to the sedimentation method.

The porous individual units preferably have an intra-particle porosity determined as the pore volume per unit particle volume within the range of from 20 vol.-% to 99 vol.-%, more preferably from 30 vol.-% to 70 vol.-%, even more preferable from 40 vol.-% to 60 vol.-%, such as 50 vol.-%, calculated from a mercury porosimetry measurement.

### Pharmaceutically acceptable excipients

Each coating layer and each matrix as described herein, i.e. the core coating b), more specifically the outer coating layer (b1) and the further coating layer (b2), and also the mucoadhesive coating layer comprising the mucoadhesive material (a2) or the mucoadhesive matrix comprising the mucoadhesive material (a2), can comprise one or more further conventional pharmaceutically acceptable excipient(s) selected from:
Antioxidants, examples of which include water soluble antioxidants such as ascorbic acid, sodium sulfite, metabisulfite, sodium miosulfite, sodium formaldehyde, sulfoxylate, isoascorbic acid, isoascorbic acid, cysteine hydrochloride, 1,4-diazobicyclo-(2,2,2)-octane, and mixtures thereof. Examples of oil-soluble antioxidants include ascorbyl palmitate, butylated hydroxyanisole, butylated hydroxytoluene, potassium propyl gallate, octyl gallate, dodecyl gallate, phenyl-α-naphthyl-amine, and tocopherols such as α-tocopherol.

Binders, examples of which include, but are not limited to, starches, celluloses and derivatives thereof, polyvinylpyrrolidone, sucrose, dextrose, corn syrup, polysaccharides, and gelatin. Examples of celluloses and derivatives thereof include for example, methylcellulose, hydroxypropyl cellulose, hydroxypropylmethyl cellulose.

Bulking agents, examples of which include, without limitation, microcrystalline cellulose, e.g., AVICEL PH from FMC (Philadelphia, PA). Particularly preferred is microcrystalline cellulose, e.g., AVICEL PH 200 from FMC (Philadelphia, PA).

Disintegrants, examples of which include, but are not limited to starches, e.g. sodium carboxymethyl starch or sodium starch glycolate; clays; alginates; gums; cross-linked polymers, e.g., cross-linked polyvinyl pyrrolidone or crospovidon, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum. Especially preferred is sodium starch glycolate, e.g. PRIMOJEL® from DFE-Pharma (Goch, Germany).

Fillers, examples of which include, but are not limited to confectioner's sugar, compressible sugar, dextrates, dextrin, dextrose, lactose, mannitol, microcrystalline cellulose, powdered cellulose, sorbitol, sucrose and talc.

Glidants and lubricants, examples of which include, but are not limited to, colloidal silica, mesoporous silica, magnesium trisilicate, starches, talc, glycerylmonostearate, tribasic calcium phosphate, magnesium stearate, aluminum stearate, calcium stearate, magnesium carbonate, magnesium oxide and polyethylene glycol. Especially preferred is glycerylmonostearate.

Surfactants, examples of which include, but are not limited to, fatty acid and alkyl sulfonates; benzethonium chloride, e.g., HYAMINE 1622 from Lonza, Inc. (Fairlawn, NJ); polyoxyethylene sorbitan fatty acid esters, e.g., the TWEEN Series from Uniqema (Wilmington, DE); and natural surfactants, such as sodium taurocholic acid, 1-palmitoyl-2-Sn-glycero-3-phosphocholine, lecithin and other phospholipids.

Plasticizers, examples of which include, but are not limited to, benzoates, organophosphates, citrates, adipates, sebacates, maleates, and specific examples thereof include, but are not limited to, triethyl citrate, dibutylsebacate, preferably triethyl citrate.

The optional one or more further pharmaceutically acceptable excipient(s) is (are) usually included in a respective coating layer or matrix in an amount of up to 50 wt.-%, preferably up to 40 wt.-%, more preferably up to 30 wt.-%, based on the total weight of the respective coating layer or matrix.

Moreover, the core (a) can comprise one or more of the pharmaceutical excipients as described above. Usually, the optional one or more further pharmaceutically acceptable excipient(s) is (are) included in the core (a) in an amount of up to 50 wt.-%, preferably up to 40 wt.-%, more preferably up to 30 wt.-%, based on the total weight of the core (a).

### Method for preparing the multiple unit dosage form of the present invention

The present invention also relates to a method of preparing the multiple unit dosage form of the present invention. In general, the preparation of the multiple unit dosage form of the present invention comprises the following steps:
i) loading or coating the individual core units with the pharmaceutically active ingredient (a1),
ii) coating each individual core unit loaded or coated with the pharmaceutically active ingredient (a1) with the mucoadhesive material (a2), or
   mixing the individual core units with the mucoadhesive material (a2) to form a matrix in which the individual core units are glued together by the mucoadhesive material (a2),
iii) forming a core (a) comprising the individual core units,
iv) coating the resulting core with the core coating (b).

The mucoadhesive material can be applied in step ii) by means of coating the individual units with a mucoadhesive coating layer comprising the mucoadhesive material (a2) or by means of mixing the individual units with a mucoadhesive material to from a matrix comprising the mucoadhesive material (a2), wherein mucoadhesive coating layer and mucoadhesive matrix, respectively, are obtained by mixing the mucoadhesive material (a2) with the further ingredients of the coating layer/matrix, using methods known in the art.

If the individual core units represent individual crystals of the active ingredient (a1), step i) is of course redundant, and the crystals of the active ingredient (a2) are directly covered with the mucoadhesive material (a2), e.g. by coating the crystals with a mucoadhesive coating layer or including them into a mucoadhesive matrix comprising the mucoadhesive material (a2), using by methods known in the art.

Exemplarily, the process can be carried out as follows:
(i) The loading of individual units, e.g. granules, pellets or functionalized calcium carbonate seed cores, with the pharmaceutically active agent, e.g. metronidazole, can e.g. be achieved by dispersing the individual units in a solution of the active ingredient in a solvent, such as e.g. ethanol or acetone, stirring the resulting dispersion, and evaporate the solvent to obtain individual units loaded with the pharmaceutically active agent.
   Alternatively, the active ingredient can also be applied as a coating, e.g. by means of layering using a fluid-bed coater or spray-drying.
(ii) Coating of the individual units loaded with the pharmaceutically active ingredient obtained in step (i) with a mucoadhesive coating layer comprising the mucoadhesive material (a2) can e.g. be achieved by means of layering using a fluid-bed coater, spray-drying, dry powder coating, suspension polymerization, ionic gelation, emulsion-solvent evaporation, supercritical fluid techniques, and fluidized-bed technologies.
   The following precipitation method can particularly be used for the application of an outer mucoadhesive coating layer comprising the mucoadhesive material (a2). In this precipitation method, the individual units loaded with the pharmaceutically active agent are mixed with a solution of the mucoadhesive material, e.g. a chitosan solution e.g. in diluted acetic acid and stirred. The pH is adjusted to about 5. After dispersion of the individual units, NaOH is added in order to adjust the pH to up to 7 while maintaining stirring. The resulting individual units comprising the pharmaceutically active ingredient (a1) and coated with a mucoadhesive coating layer comprising the mucoadhesive material (a2) can be separated from the aqueous phase by means of filtration or centrifugation, followed by drying under reduced pressure (i.e. below normal pressure of 1.01325 bar) at elevated temperatures of up to 80°C, preferably up to 70 °C, more preferably up to 60 °C. The solution can optionally comprise one or more pharmaceutically acceptable excipients as described above in order to obtain a coating further comprising pharmaceutically acceptable excipients.
   In a preferred embodiment of the present invention, the mucoadhesive material is applied by spraying a polymer solution onto the drug loaded carrier by means of a fluidized bed coater.
(iii) The individual units obtained in step (ii) are then compressed into a tablet core, using e.g. a tablet press as commonly known in the art and used by the skilled person to press tablets. Alternatively, the individual units obtained in step (ii) are filled into a capsule such as a hard-shell capsules in order to form the core a) of the multiple unit dosage form of the present invention. Step (iii) can be carried out in the presence of one or more pharmaceutically acceptable excipients as described above with which the individual units are blended before compressing them into a tablet core or filling them in a capsule.
(iv) The core obtained in step (iii) is then coated with the core coating (b). In general, the coating of the core with the core coating (b) can be conducted by usual methods known to the person skilled in the art, such as e.g. drum-coater or fluidized bed techniques. Specific methods for applying the core coating to the core are e.g. also described in WO2013/164315. The coating may also be applied together with one or more pharmaceutical excipients as described above.

### Final multiple unit dosage form

The multiple unit dosage form of the present invention is an oral dosage form being in the form of a tablet, granule, mini-tablet, pellet or a capsule, which is intended for oral administration to a patient.

The multiple unit dosage form of the present invention typically comprises 0.1 mg to 1000 mg, preferably 1 mg to 750 mg, even more preferably 5 mg to 500 mg, even more preferably 10 mg to 200 mg, even more preferably 20 mg to 150 mg, and even more preferably 40 mg to 100 mg of the pharmaceutically active ingredient.

### Multiple unit dosage form for use

The multiple unit dosage form as described above is for use in a method of treatment of inflammatory disorders, gastrointestinal disorders, such as inflammatory disorders of the gastrointestinal tract including Crohn's disease and ulcerative colitis, inflammatory bowel disease, constipation, diarrhea, infection, and carcinoma, particularly colon or colorectal cancer. Particular disorders to be treated include states of arthritis in general, rheumatoid arthritis, osteoarthritis, reactive arthritis, juvenile arthritis; psoriasis incl. psoriatic arthritis; inflammatory bowel disease, including Crohn's disease, ulcerative colitis incl. proctitis, sigmoiditis, left-sided colitis, extensive colitis and pancolitis, undetermined colitis, microscopic colitis incl. collagenous and lymphocytic colitis, colitis in connective tissue disease, diversion colitis, colitis in diverticular disease, eosinophilic colitis, pouchitis, and clostridium difficile infections. Most preferably, the antibody or functional fragment of the invention is used to treat an inflammatory bowel disease, in particular Crohn's disease, ulcerative colitis or microscopic colitis. The Crohn's disease may be ileal, colonic, ileocolonic or isolated upper Crohn's disease (gastric, duodenal and/or jejunal) including non-stricturing/non-penetrating, stricturing, penetrating and perianal disease behavior, allowing any combination of localization and disease behavior of any of the above mentioned. The ulcerative colitis may be ulcerative proctitis, proctosigmoiditis, left-sided colitis, pan-ulcerative colitis and pouchitis.

The present invention shall be illustrated in the following examples.

### Examples

### Example 1 - Mucoadhesive particle preparation

### Particle preparation

For preparation of mucoadhesive microparticles, a lab-scale fluidized-bed equipment (Strea-1, Aeromatic Fielder, Switzerland) with top-spray configuration was used. A spray nozzle with an orifice diameter of either 0.5 or 0.8 mm was used (Schlick, Germany). The spray rate was controlled using a peristalsis pump and a balance. The mucoadhesive microparticles were prepared in two steps: 1) drug loading of the porous microcarrier functionalized calcium carbonate (FCC, Omyapharm-OG) with the model drug metronidazole benzoate (MBZ) dissolved in a mixture of ethanol and acetone co-loaded with a binder polymer (polyvinylpyrrolidone), and 2) spray coating of the drug-loaded carrier particles with a chitosan solution.

Three different mucoadhesive formulations, and one non-mucoadhesive control formulation containing ethylcellulose instead of chitosan were prepared. MMW-5 and MMW-10 particles containing 5% and 10% (w/w) MMW chitosan, respectively, were prepared to evaluate the optimal chitosan content in terms of mucoadhesion. LMW-5 particles containing 5% (w/w) LMW chitosan was an optimized formulation in terms of higher drug load and easier manufacturability. The viscosity of the spray solution is lower for LMW chitosan than for MMW chitosan (20 - 300 cps vs. 200 - 800 cps, 1% (w/w) in 1% acetic acid), which results in decreased droplet size and reduced risk of nozzle blocking, leading to an overall improved coating quality. The fluidized-bed process parameters of the drug-loading and chitosan-coating batches are summarized in Table 1 and 2, respectively.

### Drug loading

Two drug-loading batches were prepared according to Table 1. For preparation of PEG-MBZ-FCC particles, PEG 3000, MBZ, and FCC were used at a ratio of 28.6:28.6:42.8 (w/w). PVP-MBZ-FCC particles were prepared at a higher drug load using PVP K-25, MBZ, and FCC at a ratio of 37.5:37.5:25 (w/w). The drug solution consisted of 10% MBZ (w/w), and 10% polymer (w/w) dissolved in a mixture of acetone and ethanol (60:40, v/v). The drug solutions were sprayed completely to reach the desired drug loads. The product was dried by fluidizing for 30 min at 40°C, and by storing overnight in a vacuum oven set to 40° C. The dried product was kept in closed jars and stored at room temperature.

### Chitosan granulation

Mucoadhesive granulation using MMW chitosan were applied on drug-loaded PEG-MBZ-FCC particles to a final chitosan content of 5% and 10% (w/w, MMW-5 and MMW-10, respectively). The granulation solution was prepared by suspending 1.5% chitosan (w/v) in purified water and adding 1.5% acetic acid (w/v). After stirring for 12 h, the pH was adjusted to pH 5.8 using NaOH 1 M, and then the solution was passed through a metal sieve with a mesh size of 90 µm (Retsch, Switzerland). To reach the desired chitosan contents of 5% and 10% (w/w), the chitosan solutions (250 ml and 500 ml, respectively) were sprayed with a spray rate set to 1-1.5 g/min. The bottom diameter of the fluidizing chamber was slightly reduced to 55 mm to process smaller quantities of particles. The optimized LMW-5 particles containing 5% (w/w) LMW chitosan were prepared using drug-loaded PVP-MBZ-FCC particles. A stainless steel chamber with 100 mm bottom diameter was used. An aqueous solution of LMW chitosan (1%, w/v) was prepared in 620 ml diluted acetic acid (10%, w/v) by stirring for 12 h and filtering through a metal sieve with a mesh size of 90 µm.

### Evaluation

Only granules containing chitosan showed a significant mucoadhesion described by longer retention of granules on the mucosa after a flow was initiated, using an ex-vivo porcine colonic mucosa.

**Table 1: Fluidized-bed process parameters used for the two drug loading batches PEG-MBZ-FCC and PVP-MBZ-FCC.**

| | PEG-MBZ-FCC | PVP-MBZ-FCC |
|---|---|---|
| FCC (g) | 180 | 96 |
| MBZ (g) | 120 | 144 |
| Polymer (g) | 120 | 144 |
| Co-loaded polymer | PEG 3000 | PVP K-25 |
| Theoretical drug load (%, w/w) | 28.6 | 37.5 |
| Inlet temperature (° C) | 50 | 50 |
| Air volume (level) | 2-3 | 2-3 |
| Atomization pressure (bar) | 0.8 | 0.8 |
| Spray rate (g/min) | 5 | 5 |
| Spray nozzle orifice diameter (mm) | 0.8 | 0.8 |
| Process time (h) | 4 | 5 |

**Table 2: Fluidized-bed process parameters for mucoadhesive coating with MMW and LMW chitosan on drug-loaded PEG-MBZ-FCC and PVP-MBZ-FCC particles, respectively.**

| | MMW-5 | MMW-10 | LMW-5 | EC-5 |
|---|---|---|---|---|
| MBZ-loaded FCC (g) | 70 | 70 | 120 | 152 |
| Chitosan coating (%, w/w) | 5 | 10 | 5 | - |
| Theoretical drug load (%, w/w) | 27.14 | 25.71 | 35.63 | 32.39 |
| Inlet temperature (° C) | 50 | 50 | 50 | 40 |
| Air volume (level) | 2-4 | 2-4 | 2-4 | 2-4 |
| Atomization pressure (bar) | 0.8 | 0.8 | 0.8 | 0.8 |
| Spray rate (g/min) | 1-1.5 | 1-1.5 | 5 | 2.5 |
| Spray nozzle orifice diameter (mm) | 0.5 | 0.5 | 0.8 | 0.8 |
| Process time (h) | 4 | 8 | 2 | 1 |

### Example 2 - Mucoadhesive particle preparation, capsule filling and coating

### Drug loading

Drug-loading of functionalized calcium carbonate (FCC) particles was conducted at a higher drug loading using polyvinylpyrrolidone (PVP K-25), metronidazole benzoate (MBZ), and FCC at a ratio of 37.5:37.5:25 (w/w). The drug solution consisted of 10% MBZ (w/w), and 10% PVP (w/w) dissolved in a mixture of acetone and ethanol (60:40, v/v). The drug solution were sprayed on the FCC particles in order to reach the desired drug loads. The inlet air temperature was 40°C, atomizing pressure 0.8 bar, air volume set to 2-3 and flow rate of 20-25 rpm (3.0 x 1.0 mm tubing). The product was dried by fluidizing for 30 min at 40°C.

### Chitosan granulation

Mucoadhesive granulation using low molecular weight chitosan was conducted on loaded PVP-MBZ-FCC particles to a final chitosan content of approximately 5% w/w. The granulation solution was prepared by suspending 1.0 % chitosan (w/v) in purified water and adding 10% acetic acid (w/v). After stirring for 12 h, the solution was passed through a sieve to reach the desired chitosan contents of 5% (w/w). The chitosan solution was sprayed completely to reach the desired loading. The inlet air temperature was 45-50°C, atomizing pressure 0.8 bar, air volume set to 3-5 and flow rate of 10-15 rpm (3.0 x 1.0 mm tubing). The product was dried by fluidizing for 30 min at 40°C.

### Preparation of a Capsule Core

A predetermined amount, based on the final target metronidazole benzoate dose, of mucoadhesive granules as obtained above were mixed with further pharmaceutically acceptable excipients such as colloidal silica and microcrystalline cellulose, and the resulting mixture was filled into hard-shell capsules. For a final dose of 100 mg of metronidazole benzoate, size 00 hard-shell capsules were used.

### Capsule banding

Filled and closed capsules were then banded with a gelatin solution to cover the area between the capsule body and capsule cap. Approximately 5 mg gelatin was applied per capsule.

### Coating the Capsule Core

### Isolation layer

The isolation layer was formed from a mixture of HPMC and 20% macrogol 6000 (PEG), based on dry polymer weight.

The HPMC was dissolved in water under magnetic stirring and then PEG 6000 was added to form a coating preparation. The coating preparation was sprayed onto banded hard-gelatin capsules using a perforated pan coating machine to achieve a coating amount of 3 mg polymer/cm².

The coating parameters were as follows: spray rate 20 g/min/kg capsules cores, atomizing pressure 0.4 bar, and inlet air temperature 45-55°C.

### Inner coating

The inner layer was applied to the isolation layer coated capsules from an aqueous preparation of Eudragit^{®} S 100, where the pH was adjusted to pH 8. The composition of the inner layer also included 70% of triethyl citrate (based on dry polymer weight), 1% potassium dihydrogen phosphate (based on dry polymer weight), 10% glyceryl monostearate (GMS) (based on dry polymer weight) and 40% polysorbate 80 (based on GMS weight). The pH was adjusted using 1 M NaOH until the pH 8 was obtained.

Potassium dihydrogen phosphate and triethyl citrate were dissolved in distilled water, after which a dispersion of Eudragit® S 100 was added under mechanical agitation. The pH was then adjusted to pH 8 with 1 M NaOH and the solution was left mixing for 1 hour.

A GMS emulsion was prepared at a concentration of 10% w/w. Polysorbate 80 (40% based on GMS weight) was dissolved in distilled water followed by dispersion of GMS. This preparation was then heated to 75°C for 15 minutes under strong magnetic stirring in order to form the emulsion. The emulsion was cooled to room temperature under stirring.

The GMS emulsion was added to the neutralized Eudragit^{®} S solution to form an inner layer coating preparation which was coated onto the isolation layer coated capsules using a perforated pan coating machine until the coating amount reached 5 mg polymer/cm² to form inner layer coated capsules.

The coating parameters were as follows: spraying rate 20 g/min/kg capsules, atomizing pressure 0.4 bar and inlet air temperature 45-55°C.

### Outer coating

The outer coating layer was applied from a mixture of aqueous starch dispersion and an organic Eudragit® S 100 solution.

The aqueous starch dispersion was prepared by dispersing maize starch into butan-1-ol, followed by water, under magnetic stirring. The ratio of maize starch:butan-1-ol was 1:1. The resulting dispersion was heated to boiling and then cooled under stirring overnight. The % solids content of the cooled preparation was calculated based on the final weight of the dispersion (considering the evaporation during heating).

The organic Eudragit® S 100 solution was prepared by dissolving Eudragit® S 100 in 96% ethanol under high speed stirring. The final solution contained about 6% polymer solids. The starch dispersion was added dropwise to the Eudragit® S 100 solution to obtain a ratio of starch:Eudragit® S of 50:50. The mixture was mixed for 2 hours and 20% triethyl citrate (based on total polymer weight) and 5% glyceryl monostearate (GMS) (based on total polymer weight) were added and mixed for further 2 hours.

The GMS was added in the form of a dispersion prepared at a concentration of 5% w/w. Polysorbate 80 (40% based on GMS weight) was dissolved in distilled water followed by dispersion of the GMS. This dispersion was then heated to 75 °C for 15 minutes under strong magnetic stirring in order to form an emulsion. The emulsion was cooled at room temperature and under stirring.

A pigment suspension in ethanol containing iron oxide red (6.09 mg per capsule) and iron oxide yellow (1.05 mg per capsule) prepared under high shear homogenization for 10 minutes was added to the final coating suspension.

The final preparation was coated on to the capsule cores, using a perforated pan coating machine until a coating having 10 mg total polymer/cm² was obtained. The spray coating parameters were as follows: spraying rate 20 g/min/kg capsules, atomizing pressure 0.4 bar and inlet air temperature 45-55 °C. The coated capsules were then polished with 1 mg PEG 6000 per capsule and dried overnight at 40°C.

### Comparative Example 1 - Non-mucoadhesive particle preparation, capsule filling and coating

### Drug loading

Drug-loading of functionalized calcium carbonate (FCC) particles was conducted at a higher drug loading using polyvinylpyrrolidone (PVP K-25), metronidazole benzoate (MBZ), and FCC at a ratio of 37.5:37.5:25 (w/w). The drug solution consisted of 10% MBZ (w/w), and 10% PVP (w/w) dissolved in a mixture of acetone and ethanol (60:40, v/v). The drug solution was sprayed on the FCC particles to reach the desired drug loads. The inlet air temperature was 40°C, atomizing pressure 0.8 bar, air volume set to 2-3 and flow rate of 20-25 rpm (3.0 x 1.0 mm tubing). The product was dried by fluidizing for 30 min at 40°C,

### Ethylcellulose granulation

The granulation of metronidazole benzoate particles was performed by blending ethylcellulose to achieve a final ethylcellulose content of 5% in the final granules. Ethylcellulose is often used as control to evaluate mucoadhesion of dosage forms as this excipient has very low mucoadhesive properties. The granulation solution was prepared by dissolving polyvinylpyrrolidone (PVP K-25) in purified water. The binding solution was sprayed completely. The inlet air temperature was 45-50°C, atomizing pressure 0.8 bar, air volume set to 3-5 and flow rate of 10-15 rpm (3.0 x 1.0 mm tubing). The product was dried by fluidizing for 30 min at 40°C.

### Preparation of a Capsule Core

A predetermined amount, based on final target metronidazole benzoate dose, of **non-mucoadhesive** granules as obtained above were mixed with further pharmaceutically acceptable excipients, such as colloidal silica and microcrystalline cellulose, and the resulting mixture was filled in hard-shell gelatin capsules. For a final dose of 100 mg of metronidazole benzoate, size 00 hard-shell capsules were used.

### Capsule banding

Filled and closed capsules were then banded with a gelatin solution to cover the area between the capsule body and capsule cap. Approximately 5 mg gelatin was applied per capsule. After banding, capsules were let drying at room temperature.

### Coating the Capsule Core

### Isolation layer

The isolation layer was formed from a mixture of HPMC and 20% macrogol 6000 (PEG), based on dry polymer weight.

The HPMC was dissolved in water under magnetic stirring and then PEG 6000 was added to form a coating preparation. The coating preparation was sprayed onto banded hard-getatin capsules using a perforated pan coating machine to achieve a coating amount of 3 mg polymer/cm².

The coating parameters were as follows: spray rate 20 g/min/kg capsules cores, atomizing pressure 0.4 bar, and inlet air temperature 45-55°C.

### Inner coating

The inner layer was applied to the isolation layer coated capsules from an aqueous preparation of Eudragit^{®} S 100, where the pH was adjusted to pH 8. The composition of the inner layer also included 70% of triethyl citrate (based on dry polymer weight), 1 % potassium dihydrogen phosphate (based on dry polymer weight), 10% glyceryl monostearate (GMS) (based on dry polymer weight) and 40% polysorbate 80 (based on GMS weight). The pH was adjusted using 1 M NaOH until the pH 8 was obtained.

Potassium dihydrogen phosphate and triethyl citrate were dissolved in distilled water, after which a dispersion of Eudragit^{®} S 100 was added under mechanical agitation. The pH was then adjusted to pH 8 with 1 M NaOH and the solution was left mixing for 1 hour.

A GMS emulsion was prepared at a concentration of 10% w/w. Polysorbate 80 (40% based on GMS weight) was dissolved in distilled water followed by dispersion of GMS. This preparation was then heated to 75°C for 15 minutes under strong magnetic stirring in order to form the emulsion. The emulsion was cooled to room temperature under stirring.

The GMS emulsion was added to the neutralised Eudragit^{®} S solution to form an inner layer coating preparation which was coated onto the isolation layer coated capsules using a perforated pan coating machine until the coating amount reached 5 mg polymer/cm² to form inner layer coated capsules.

The coating parameters were as follows: spraying rate 20 g/min/kg capsules, atomizing pressure 0.4 bar and inlet air temperature 45-55°C.

### Outer coating

The outer coating layer was applied from a mixture of aqueous starch dispersion and an organic Eudragit® S 100 solution.

The aqueous starch dispersion was prepared by dispersing maize starch into butan-1-ol, followed by water, under magnetic stirring. The ratio of maize starch:butan-1-ol was 1:1:. The resulting dispersion was heated to boiling and then cooled under stirring overnight. The % solids content of the cooled preparation was calculated based on the final weight of the dispersion (considering the evaporation during heating).

The organic Eudragit® S 100 solution was prepared by dissolving Eudragit® S 100 in 96% ethanol under high speed stirring. The final solution contained about 6% polymer solids. The starch dispersion was added dropwise to the Eudragit® S 100 solution to obtain a ratio of starch:Eudragit® S of 50:50. The mixture was mixed for 2 hours and 20% triethyl citrate (based on total polymer weight) and 5% glyceryl monostearate (GMS) (based on total polymer weight) were added and mixed for further 2 hours.

The GMS was added in the form of a dispersion prepared at a concentration of 5% w/w. Polysorbate 80 (40% based on GMS weight) was dissolved in distilled water followed by dispersion of the GMS. This dispersion was then heated to 75 °C for 15 minutes under strong magnetic stirring in order to form an emulsion. The emulsion was cooled at room temperature and under stirring.

A pigment suspension in ethanol containing iron oxide red (6.09 mg per capsule) and iron oxide yellow (1.05 mg per capsule) prepared under high shear homogenization for 10 minutes was added to the final coating suspension.

The final preparation was coated on to the capsule cores, using a perforated pan coating machine until a coating having 10 mg total polymer/cm² was obtained. The spray coating parameters were as follows: spraying rate 20 g/min/kg capsules, atomizing pressure 0.4 bar and inlet air temperature 45-55 °C. The coated capsules were then polished with 1 mg PEG 6000 per capsule and dried overnight at 40°C.

### Example 3 - Comparison of in-Vitro Release Properties of Dosage Forms obtained in Example 2 and Comparative Example 1

Example 2 and Comparative Example 1 are examples of final coated dosage forms comprising either mucoadhesive (Example 2) or non-mucoadhesive granules (Comparative Example 1).

Both embodiments were tested with respect to their release properties simulating different conditions including simulated gastric conditions (2h in 0.1 N HCl), simulated small intestinal conditions (Hanks buffer pH 6.8), and simulated ileo-colonic conditions (Krebs buffer pH 7.4). The results are summarized in Table 3.

As may be taken from Table 3, both embodiments according to Example 2 and Comparative Example 1 are fully resistant to gastric simulated conditions (2h in 0.1 N HCl) (Table 3, entry 1).

The embodiments according to Example 2 and Comparative Example 1 are also fully resistant to simulated small intestinal conditions (Hanks buffer pH 6.8) for at least 240 min (small intestinal transit time). In case of the dosage form of Example 2, the lagtime for a 5% release was 540 min, whereas it was 290 min for comparative example 1 (Table 3, entry 2).

In simulated ileo-colonic conditions (Krebs buffer pH 7.4), both embodiments according to Example 2 and Comparative Example 1 start to release within 2 hours. For the dosage form of Example 2, the lagtime for a 5% release was 130 min, and 100 min for the dosage form of Comparative Example 1 (Table 3, entry 3).

The embodiment in Example 2 was designed to provide sustained drug release under ileo-colonic conditions after capsule opening (80% release after 1420 minutes, Table 3, entry 4), whereas the embodiment in Comparative Example 1 was designed to allow immediate and complete release under ileo-colonic conditions after capsule opening (80% release after 130 minutes, Table 3, entry 4).

**Table 3: In-Vitro release data for dosage forms of Example 2 and comparative Example 1**

| **Entry** | **Condition** | **Example 2** | **Comparative Example 1** |
|---|---|---|---|
| 1 | **Acid resistance (2h 0.1 N HCl)** | 100% | 100% |
| 2 | **Lagtime (5%) release in Hanks buffer pH 6.8 (min)** | 540 min | 290 min |
| 3 | **Lagtime (5%) release in Krebs buffer pH 7.4 (min)** | 130 min | 100 min |
| 4 | **Lagtime release (80%) in Krebs buffer pH 7.4 (min)** | 1420 min | 130 min |

### Dissolution in Hanks buffer pH 6.8

*In vitro* dissolution studies are performed on a USP type II apparatus using a paddle speed of 50 rpm and a media temperature of 37 ± 0.5 °C. Capsules are first tested in 0.1 M HCl for 2 h followed by in Hanks buffer (pH 7.4), which closely resembles the electrolyte composition of the human small intestinal fluid. The pH of the buffer is stabilized at 6.8 ± 0.05 by continuously sparging with 5% CO₂ / 95% O₂. Absorbance measurements are taken at regular intervals, The composition per liter of Hanks buffer is 0.06 g of KH₂PO₄, 0.06g Na₂HPO₄·2H₂O, 8 g of NaCl, 0.4 g KCI, 0.2 g MgSO₄·7H₂O, 0.14 g CaCl₂·2H₂O and 0.350 g NaHCO₃.

### Dissolution in Krebs buffer pH 7.4

*In vitro* dissolution studies are performed on a USP type II apparatus using a paddle speed of 50 rpm and a media temperature of 37 ± 0.5 °C. Capsules are first tested in 0.1 M HCl for 2 h followed by in Krebs buffer (pH 7.4), which closely resembles the electrolyte composition of the human ileo-colonic fluid. The pH of the buffer is stabilized at 7.4 ± 0.05 by continuously sparging with 5% CO₂ / 95% O₂. Absorbance measurements are taken at regular intervals, The composition per liter of Krebs buffer is 0.16 g of KH₂PO₄, 6.9 g of NaCl, 0.35 g KCI, 0.29 g MgSO₄·7H₂O, 0.376 g CaCl₂·2H₂O and 2.1 g NaHCO₃.

## Claims

1. A multiple unit dosage form comprising
(a) a core and
(b) a core coating which is a coating which completely covers the core,
wherein the core comprises a plurality of individual units, each unit comprising
(a1) at least one pharmaceutically active ingredient, and wherein each unit is covered by
(a2) at least one mucoadhesive material,
wherein the core coating (b) comprises at least one coating layer, namely an outer coating layer (b1),
wherein the outer coating layer (b1) of the core coating (b) is an enteric coating layer which is substantially insoluble in the stomach of a patient and soluble in the intestine of a patient.

2. The multiple unit dosage form according to claim 1, wherein the core coating (b) comprises a further coating layer (b2),
wherein said further coating layer (b2) is between the core (a) and the outer coating layer (b1), and wherein said further coating layer (b2) comprises a polymeric material which is soluble in intestinal fluid or gastric fluid and which is selected from the group consisting of a polycarboxylic acid polymer that is at least partially neutralized and a non-ionic polymer, each in combination with at least one additive selected from a buffer agent and/or a base and mixtures thereof.

3. The multiple unit dosage form according to claim 2, wherein the further coating layer (b2) of the core coating (b) is an alkaline layer, which means that the layer material when dissolved in 10 times its weight of water provides a pH value above the pH threshold at which the enteric polymer in the outer coating (b1) dissolves.

4. The multiple unit dosage form according to any of the preceding claims, wherein the at least one mucoadhesive material (a2) comprises chitosan or alginate, preferably chitosan.

5. The multiple unit dosage form according to any of the preceding claims, wherein the at least one mucoadhesive material (a2) is present in a mucoadhesive controlled release coating layer covering the individual units, wherein the active ingredient is released over a time period of at least 30 minutes in simulated intestinal fluid when tested under the following conditions:
USP type II apparatus using a paddle speed of 50 rpm and a media temperature of 37 ± 0.5 °C, using first 0.1 M HCl for 2 h followed by Krebs buffer (pH 7.4), wherein the composition per liter of Krebs buffer is 0.16 g of KH₂PO₄, 6.9 g of NaCl, 0.35 g KCI, 0.29 g MgSO₄·7H₂O, 0.376 g CaCl₂·2H₂O and 2.1 g NaHCO₃,
wherein the time period of the release starts when the sample is treated with the Krebs buffer.

6. The multiple unit dosage form according to any of the preceding claims, wherein the outer coating layer (b1) of core coating (b) which is an enteric coating layer comprises a polymeric material which has a pH threshold of about pH 5 or above, i.e. which dissolves at a pH threshold of about pH 5 or above and is insoluble at more acidic pH values.

7. The multiple unit dosage form according to claim 6, wherein the outer coating layer (b1) which is an enteric coating of core coating (b) also comprises a polymeric material which is susceptible to an attack by colonic bacteria.

8. A multiple unit dosage form according to any of the preceding claims, wherein the individual units are selected from porous microparticles, granules, pellets and mini tablets, preferably pellets.

9. The multiple unit dosage form according any of the preceding claims, wherein each individual unit has a maximum outer diameter in the range of 100 µm to 1 mm, preferably in the range of 100 µm to 500 µm.

10. The multiple unit dosage form according to any of the preceding claims, wherein the core is a tablet or a hard-shell capsule, preferably a hard-shell capsule.

11. The multiple unit dosage form according to any of the preceding claims, wherein the pharmaceutically active ingredient (a1) is an anti-inflammatory agent or antibiotic.

12. The multiple unit dosage form according to any of claims 1 to 11, wherein the pharmaceutically active ingredient (a1) is selected from metronidazole, vancomycine, 5-amino-salicylic acid, budesonide, and anti-TNFs, and is preferably metronidazole.

13. The multiple unit dosage form according to any of the preceding claims, wherein the individual units of core (a) comprise an inert seed core onto which a first coating layer is coated containing the pharmaceutically active ingredient (a1) onto which a mucoadhesive coating layer comprising the mucoadhesive material (a2) is coated.

14. The multiple unit dosage form according to claim 13, wherein the seed core comprises, preferably consists of functionalized calcium carbonate (FCC) as porous drug carrier.

15. The multiple unit dosage form according to any of the preceding claims for use in the treatment of inflammatory disorders.

16. The multiple unit dosage form according to claim 15 for use in the treatment of inflammatory bowel diseases wherein the inflammatory bowel disease is preferably selected from Crohn's disease, ulcerative colitis and other kinds of colitis including.
